(19) Europäisches Patentamt
European Patent Office
Office européen des brevets

(11) Publication number: **0 259 423 B1**

(12) **EUROPEAN PATENT SPECIFICATION**

(45) Date of publication of patent specification: **21.04.93**

(51) Int. Cl.[5]: **C07C 69/90**, C07C 69/734, C09K 19/20, C09K 19/12

(21) Application number: **87901551.9**

(22) Date of filing: **20.02.87**

(86) International application number: **PCT/GB87/00132**

(87) International publication number: **WO 87/05291 (11.09.87 87/20)**

# (54) LIOUID CRYSTAL COMPOUNDS, MIXTURES AND DEVICES.

(30) Priority: **26.02.86 GB 8604739**
**20.06.86 GB 8615095**

(43) Date of publication of application:
**16.03.88 Bulletin 88/11**

(45) Publication of the grant of the patent:
**21.04.93 Bulletin 93/16**

(84) Designated Contracting States:
**AT BE CH DE FR GB IT LI NL SE**

(56) References cited:
**EP-A- 0 104 327**

(73) Proprietor: **The Secretary of State for Defence in Her Britannic Majesty's Government of the United Kingdom of Great Britain and Northern Ireland Whitehall London SW1A 2HB(GB)**

Proprietor: **THE SHANGHAI INSTITUTE OF ORGANIC CHEMISTRY OF THE CHINESE ACADEMY OF SCIENCE
345 Lingling Road
Shanghai(CN)**

(72) Inventor: **GRAY, George, William
"Glenwood" Newgate Street Cottingham
North Humberside HU16 4D7(GB)**
Inventor: **LACEY, David
19 Kildare Close
Hull, North Humberside HU8 9NN(GB)**
Inventor: **TOYNE, Kenneth, Johnson
25 Hall Road Hull
North Humberside HU6 8OW(GB)**
Inventor: **SCROWSTON, Richard, Michael
11 Saunders Croft Walkington
Beverley North Humberside(GB)**
Inventor: **HOLMES, David
Flat 2 15 Harley Street Hull
North Humberside HU2 9BE(GB)**
Inventor: **BRADSHAW, Madeline, Joan
"Underleaf" Upleadon Near Newent
Gloucester GL18 1BJ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

Inventor: **SAMRA, Anerjit**
**5 Rutland Drive Bromsgrove**
**Worcestershire B60 2PB(GB)**
Inventor: **HONG, Xi, Jun Shanghai Inst. of Org. Chem.**
**Chinese Academy of Science 345 Lingling Road**
**Shangai(CN)**

(74) Representative: **Beckham, Robert William et al**
**Defence Research Agency Intellectual Property Department DRA Farnborough**
**Farnborough, Hants. GU14 6TD (GB)**

## Description

This invention relates to liquid crystal mixtures, to compounds and to the use of compounds in ferroelectric smectic liquid crystal mixtures. The invention is particularly concerned with ferroelectric liquid crystal mixtures and compounds. The invention also relates to electro-optical devices incorporating these mixtures.

Ferroelectric liquid crystal materials utilise the ferroelectric properties of the chiral smectic phase, ie the chiral smectic C, F, G, H, I, F and K phases (hereinafter designated $S_C$* etc., the asterisk * denoting chirality). The $S_C$* phase is most commonly sought for use in electro-optical devices as it is the most fluid, and it is also desirable that the material shows a $S_A$ phase at a temperature above the $S_C$* phase, to assist in surface alignment.

Ferroelectric liquid crystal materials ideally have a low viscosity, a broad smectic liquid crystal temperature range, stability, etc, and in particular should show a high spontaneous polarisation coefficient ($P_S$, measured in $nCcm^{-2}$). Although some single component materials show these properties, it has become common practice to use a mixture which shows a smectic phase. At least one of the compounds in the mixture is optically active (chiral) so as to induce the smectic phase shown by the mixture to be chiral, especially $S_C$*. Such optically active compounds are called "chiral dopants".

According to the present invention there is provided use of a compound of Formula I for inclusion in a ferroelectric smectic liquid crystal mixture, where Formula I is:

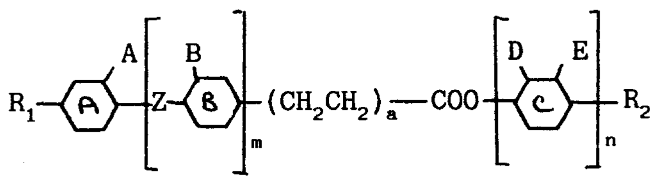

Formula I

where each of

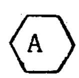 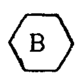 and 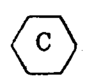

are independently selected from phenyl, trans-cyclohexyl or pyrimidyl, n is 0 or 1, m is 1, 2 or 3 (provided m + n is 1, 2 or 3), each of A, B, D and E is independently hydrogen or fluorine, $R_1$ is alkyl or alkoxy containing up to 15 carbon atoms, Z is selected from COO, OOC, $CH_2CH_2$, $OCH_2$, $CH_2O$, CH = N, N = CH, N = N, N(O) = N or a single bond; and if n is 0, $R_2$ is alkyl containing up to 15 carbon atoms and if n is 1, $R_2$ is selected from $-COOR_3$ or $-CH(CH_3)COOR_4$ where $R_3$ is optically active alkyl or n-alkyl and $R_4$ is n-alkyl and $R_3$ and $R_4$ contain up to 12 carbon atoms ; and a is 1 or 2.

In accordance with a further aspect of the invention there is provided a ferroelectric smectic liquid crystal mixture, being a mixture of compounds at least one of which is a compound of formula I above or of formula II below:

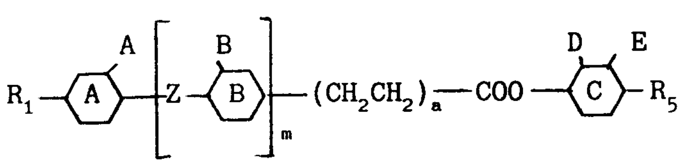

Formula II

where each of the Figs A B and C , and A, B, D, E, Z, and a are as defined in formula I above, $R_1$ is alkyl or alkoxy containing up to 15 carbon atoms, m is 1 or 2, and $R_5$ is n-alkyl or n-alkoxy, or optically active alkyl or alkoxy and contains up to 15 carbon atoms.

EPA 0104327 and JPA laid open No. 199856/1985 describe compounds of similar structure to formula I and II but exclusively for use in nematic liquid crystal mixtures. It is well known that the physics and chemistry of nematic and ferroelectric smectic liquid crystal mixtures is very substantially different, and the understanding of nematic systems has little bearing on smectic systems. The nematic liquid crystal mixtures of EPA 0104327 are also described as suitable for use in dielectric anisotropy devices. Such

devices are switched by action of an electric field on the dielectric anisotropy characteristic of the mixture. Ferroelectric smectic liquid crystal devices are switched by action of an electric field on the spontaneous polarisation characteristic of the ferroelectric smectic liquid crystal mixture. Switching times of ferroelectric smectic liquid crystal devices are of the order of microseconds, whereas switching times of nematic dielectric anisotropy devices are of the order of milliseconds.

$R_1$ maybe n-alkyl or n-alkoxy, or may be optically active.

Preferably in formulae I and II

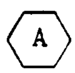 , 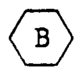 and 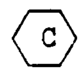

are all phenyl when present. Preferably Z is a single bond. Preferably m and a are 1 so that preferred compounds of formula I and II have formulae IA and IIA respectively;

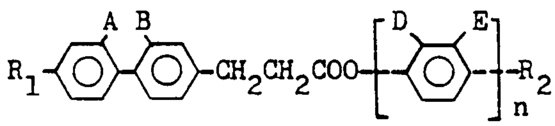

formula IA

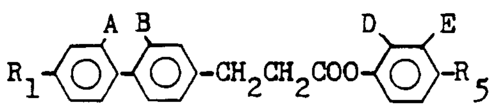

formula IIA

Preferably in formulae I, IA, II and IIA $R_1$ contains 5 or more carbon atoms, $R_3$ or $R_4$ when optically active are selected from 2-methyl butyl or 2-octyl (2-methylheptyl). Preferably A, B, D and E are either all hydrogen or else only one is fluorine.

Particularly preferred structural forms are therefore listed below:

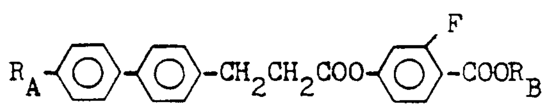

I.1

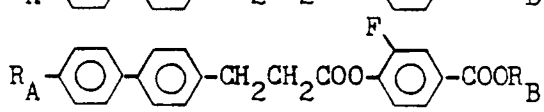

I.2

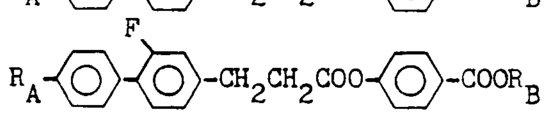

I.3

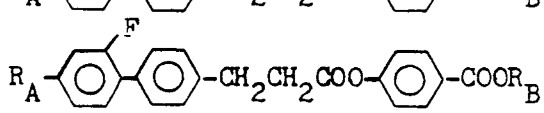

I.4

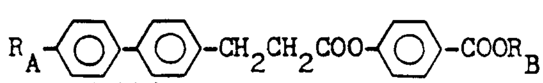

I.5

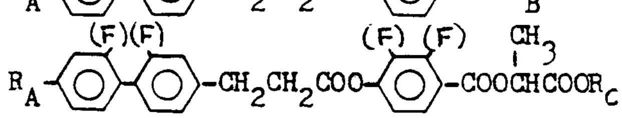

I.6

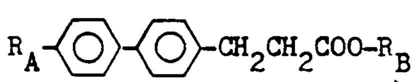

I.7

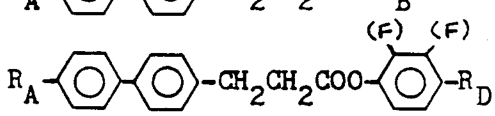

II.1

where $R_A$ is n-alkyl or n-alkoxy, or optically active alkyl or alkoxy, $R_B$ is alkyl, eg straight chain, branched or chiral,(F) indicates that the structure may carry one fluorine substituent at one of the

substitution positions indicated in the structure, $R_C$ is n-alkyl, $R_D$ is n-alkyl, n-alkoxy or optically active alkyl or alkoxy. Each of $R_A$, $R_B$, $R_C$ and $R_D$ contains up to 15 carbon atoms.

The compound of formula I or II may be in an optically active, racemic or non-optically active form, and the compound may be used in a ferroelectric smectic liquid crystal mixture in any of these forms. Optically active groups may be present in their racemic form.

Such a ferroelectric smectic mixture will normally consist of one or more racemic or non-optically active compounds, which may include one or more racemic or non-optically active compounds of formula I or II, together with one or more optically active compounds, which may be compounds of formula I or II.

The non-optically active or racemic components of such a mixture are often referred to individually or collectively as "smectic hosts". A wide range of smectic hosts is known, but some particularly preferred hosts are:

(a) The compounds disclosed in WO 86/04327, eg having a formula:

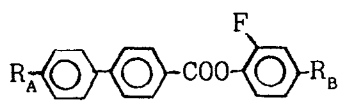

where $R_A$ is n-alkyl or n-alkoxy and $R_B$ is n-alkyl, or mixtures thereof, especially eutectic mixtures.

(b) The terphenyls disclosed in EPA 84304894-3 eg having a formula:

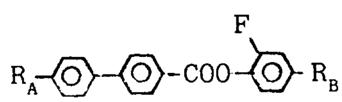

where $R_A$ and $R_B$ are independently selected from n-alkyl and n-alkoxy, or mixtures thereof, especially eutectic mixtures.

Other known examples of smectic hosts include compounds of the following general formulae:

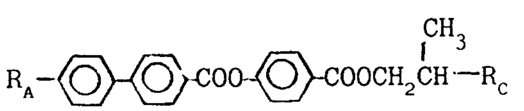

(racemic)

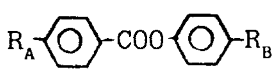

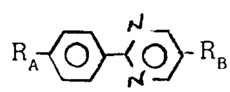

where $R_A$ and $R_B$ are independently selected from n-alkyl and n-alkoxy and $R_C$ is n-alkyl, preferably ethyl.

The mixture may also contain other optically active compounds which are miscible with smectic materials, for example the lactic acid derivatives disclosed in WO 86/02937, eg

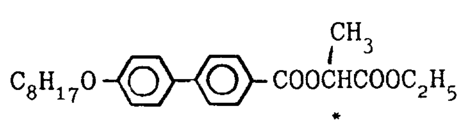

or the compounds disclosed in EPA 0110299, eg:

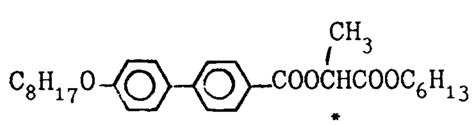

or the terpenoid derivatives disclosed in WO 86/04327, or terphenyl compounds having a formula:

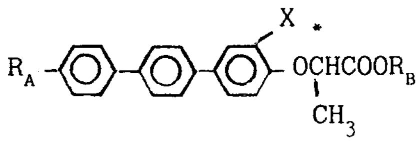

where $R_A$ contains from 1 to 12 carbon atoms and is n-alkyl or n-alkoxy, and X is fluorine or chlorine, and $R_B$ is n-alkyl containing 1 to 12 carbon atoms.

If the mixture includes more than one optically active compound eg a compound of formula I or II plus one or more of the optically active compounds referred to above, or two ore more of the optically active compounds referred to above, helical twist senses of the S* phase induced by these compounds may be the same or opposite, eg by the use of both (+) and (-) optically active compounds. It is often desirable to include compounds which induce opposite twist senses of the S* phase in order to increase the helical pitch length. It is desirable that each optically active compound in the mixture induces the same sense of Ps in the mixture.

The mixture may also contain other known additives to improve the properties. eg Ps, Sc phase breadth, viscosity etc or to induce the appearance of an $S_A$ phase of a temperature above the $S^*_C$ to assist alignment. An example of a class of compounds which may be used to broaden the Sc phase is:

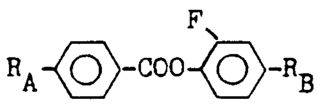

where $R_A$ and $R_B$ are independently $C_{1-12}$ n-alkyl or n-alkoxy.

The mixture may also contain pleochroic dyes.

Typically but not exclusively a ferroelectric smectic liquid crystal mixture of the invention will have the following composition:

| | |
|---|---|
| One or more smectic host compounds, which may include one or more non-optically active or racemic compounds of formula I or II. | 50 - 99 wt % |
| One or more optically active compounds (dopants) which may include one or more compounds of formula I or II. | 1 - 50 wt % (especially up to ca. 20 wt %) |
| Additives and pleochroic dyes if present. | 0 - 20 wt % |

The total being 100 weight%. The nature and relative proportions of the various components of a liquid crystal material of the invention will depend upon the use for which the material is intended, and some experimentation may be necessary to suit a particular requirement, but the basic principles of mixing and assessment of such materials is well known in the field.

The liquid crystal materials of the invention may be used in any of the known types of ferroelectric smectic liquid crystal display device, eg the "Clark-Lagerwall Device" described in Appl. Phys Lett (1980), 36 899 and in Recent Developments in Condensed Matter Physics (19810, 4,309. The physics of this type of device and the method of constructing it are well known, and are described for example in WO 86/04327, and WO 86/02937. In practice such a device usually consists of two substrates, at least one of which is

optically transparent, electrodes on the inner surfaces of the substrates by which a voltage may be applied and a layer of the liquid crystal materials sandwiched between the substrates. It is desirable that the helical pitch length of the $S_C^*$ phase is comparable to the thickness of the material which is why long pitch mixtures are useful. The materials of the invention may be used in both the birefringence type display mode and the guest-host type display mode of the Clark-Lagerwall device. The device may for example be in the form of a clock, calculator or video screen display, and methods of making the device in this form will be well known to those skilled in the art.

A generally applicable preparative route for compounds of formulae I and II, particularly for those of formula IA and IIA is route A shown in Fig. 1. Compound (A) may be prepared by simple methods from the commercially available methoxy-cyanobiphenyl. The carboxylic acid (B) may then be esterified with the appropriate alcohol or phenol. Although shown in Fig. 1 for the preparation of a compound of formula II.2 the adaptation of the method to the preparation of other compounds of formula I and II will be apparent to one skilled in the art.

The invention will now be described by way of example only with reference to the following figures:

| | |
|---|---|
| Fig 1. | shows preparative route A. |
| Figs 2, 3 and 4. | show graphs of Ps vs T and response time vs voltage for liquid crystal mixtures of the invention. |
| Fig. 5. | shows a liquid crystal device of the invention. |

Example 1

An example of the use of Route A.

Step IA(i)

Preparation of 4-hydroxymethyl-4'-methoxy biphenyl.

4'-Methoxybiphenyl-4-carboxylic acid (2.85g., 12.5 mmol), obtained by acid hydrolysis by known procedures from commercially available 4-cyano-4'-methoxybiphenyl, was dissolved in dry tetrahydrofuran (THF) (20 ml). The solution was cooled to 0 °C and a solution of borane, $BH^3$, (l7ml, 17mmol of a 1M solution in THF) was added through a syringe under nitrogen, during about 20 minutes. The ice bath was removed and replaced by a water bath at 25 °C, whereupon stirring was continued for 1 hr (reaction complete by tlc). The excess of borane was destroyed by careful addition of l0ml of a 1:1 mixture of THF:water. The reaction mixture was treated with chloroform and water, shaken briefly. and the organic layer separated off. After drying ($MgSO_4$) and removal of solvent, the product was crystallised from ethanol. The yield was quantitative: mpt. 162-3 °C (lit mpt. 161 - 2 °C).

Step IA(ii)

Preparation of 4-bromomethyl-4'-methoxybiphenyl.

A solution of phosphorus tribromide (1.3 ml, 13.8 mmol) in benzene (3 ml) was added to 4-hydroxymethyl-4'-methoxy biphenyl (1.5 g, 7 mmol) dissolved in benzene (6 ml) at room temperature. The reaction mixture was then boiled for 3 hr, with separation of a yellow solid. On being cooled. the reaction mixture was poured onto ice and ether. The ethereal solution obtained after shaking (the yellow solid was not soluble) was washed with water, aqueous sodium carbonate, then water, and was dried ($MgSO_4$). Removal of solvent gave a white solid, yield: 1.7 g, 87.6% which was used in step IA(iii) below without purification.

Step IA(iii)

Preparation of 3-(4'-methoxybiphenyl-4-yl) propanoic acid.

To a solution of sodium (2.6g, 0.11 g atom) in absolute ethanol (55ml), diethyl malonate (42m1., 0.27 mol) was added to yield a clear solution which was then evaporated to dryness.

4-Bromomethyl-4'-methoxybiphenyl (16.3 g, 0.059mol) in dry benzene (125 ml) was added in one portion to the sodium salt of diethyl malonate and the mixture was heated at 75 °C for $5\frac{1}{2}$ hours, The cooled mixture was poured into water and the whole was shaken with ether. Separation of the ether layer and

washing with water were followed by drying ($MgSO_4$) and removal of the solvent. The oil obtained was hydrolysed by boiling with a mixture of 10M NaOH (55ml) and 1:1 water : ethanol (450 ml) for 2 hr. Removal of the ethanol by distillation was followed by addition of a large volume of water to obtain a solution of the sodium salt of the dicarboxylic acid. This solution was washed with ether to remove some insoluble oil. The aqueous solution was acidified (to pH 1) with conc. HCl. The precipitate of the di-acid (94 %) was filtered off and dried.

Decarboxylation was achieved by heating the white, powdered di-acid progressively to 180 °C. The product ( the mono-acid) was obtained in 91 - 92 % yield, mp 196-7 °C.

Step IA(iv)

Preparation of 3-(4'-hydroxybiphenyl-4-yl) propanoic acid.

The methyl ether from Step IA(iii) (10.24 g, 0.04 mmol) was suspended in dry dichloromethane (20 ml) and the mixture stirred as the temperature was lowered to -70 °C, when a solution of boron tri-bromide in dichloromethane (8 ml of an 0.06 M solution) was added dropwise. After stirring for 30 min. the mixture was allowed to warm to room temperature, with stirring, which was continued for 4 hr. The mixture was poured into ice water, a few drops of conc. HCl were added, and the whole was stirred overnight. The product (9.5g, 98%) mpt. 207 - 210 °C, was obtained by filtration and drying. The occurence of demethylation was checked by IR spectroscopy. The product was dificult to crystallise and was used without purification in the further alkylation reactions, Step IA(v).

Step IA(v)

Preparation of 3-(4'-alkoxybiphenyl-4-yl)propanoic acids.

3-(4'-Hydroxybiphenyl-4-yl) propanoic acid (4.84g , 0.02 mol) was dissolved in 2-methoxyethanol (145 ml) with heating; to the hot solution was added (with vigorous stirring) a solution of KOH (2.24g 0.04 mol) in water (7.5 ml) (overall mixture 95:5, 2-methoxyethanol : water). After 10 min. the bromoalkane (0.024 mol) was added and the reaction mixture heated under reflux with stirring overnight. A solution of KOH (2g) in water (20 ml) was then added and boiling was continued for two further hours.

The cooled reaction mixture was poured into a large volume (1.5 L) of water, and acidified to Congo Red with conc. HCl. The suspension of free acid was stirred for about 2 hours before the product was filtered off, washed and crystallised from 95% acetic acid.

The product acids were checked for identity and purity by IR spectroscopy, NMR spectrometry and mass spectrometry. Yields after crystallisation were 65 - 75 %.

Two examples of acids prepared in this was are characterised below:

| | |
|---|---|
| Pentyl ether | mpt. 196.7 - 197.4 °C; m/e = 312 |
| Nonyl ether | mpt. 183.5 - 184.7 °C; m/e = 368 |

Esterification

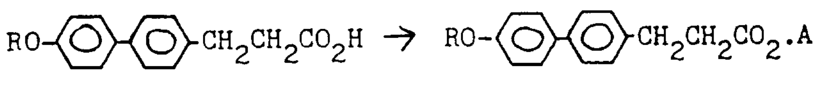

The 3-(4'-alkoxybiphenyl-4-yl) propanoic acid (0.001 mol), the phenol or alcohol (A.OH; 0.0011 mol), N,N'-dicyclohexylcarbodiimide (0.0011 mol) and 4-pyrrolidinopyridine (0.0001 mol) were mixed in dry dichloromethane (10 ml). The reaction mixture was stirred at room temperature for 15 - 20 hr, completion of reaction being checked by tlc.

Dicyclohexylurea was removed by filtration and the solution in dichloromethane was combined with dichloromethane washings of the urea derivative. Washings of the solution with water (3 x 10ml), 5% acetic acid solution (3 x 10ml) and water was followed by drying and removal of the solvent by evaporation. Yields at this stage were nearly quantitative.

Products were finally purified by a combination of crystallisation (ethanol or light petroleum bp. 40 - 60 °C) and column chromatography on 60 - 120 mesh silica gel using dichloromethane as solvent and

eluant.

The products were pure by tlc and were checked by IR, NMR and MS. Purities in excess of 99.8% were obtained by hplc and satisfactory microanalytical results were found.

Some properties of compounds of formula I and II prepared by route A are listed in tables 1 and 2. Note: unless otherwise stated A, B, D, E are hydrogen, R5 means $C_5H_{11}$-n, R5.0 means $C_5H_{11}O$-n etc.

Table 1.

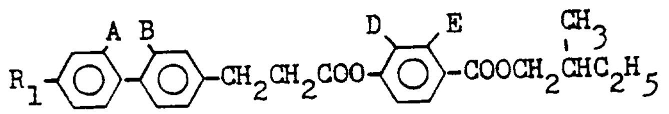

(±)

Liquid crystal transition temperatures (°)

| $R_1$ | K | $S_E$ | $S_B$ | $S_{I/F}$ | $S_C$ | $S_A$ | I |
|---|---|---|---|---|---|---|---|
| R5.0 | | | 74 | | | 104 | 129 |
| R6.0 | | 54 | 58 | | | 100 | 130 |
| R7.0 | | | | 55 | 91 | 100 | 127 |
| R8.0 | | | | 59 | 91 | 107 | 127 |
| R9.0 | | | | 67 | 83 | 108 | 126 |
| R10.0 | | | | 75 | 82 | 112 | 125 |
| R11.0 | | | | | 79 | 112 | 123 |
| R9 | | | | 35 | 72 | 74 | 95 |
| R8.0(E=F) | | | | 50 | 62 | 103 | 120 |
| R10.0(E=F) | | | | 49 | 56 | 106 | 117 |
| R12.0(E=F) | | | | | 58 | 103 | 116 |
| R8.0(D=F) | | 52 | | 57 | 81 | 84 | 110 |
| R8.0(B=F) | | | | | | 55 | 91 |
| R8.0(A=F) | | | | | | 46 | 83 |

Table 2

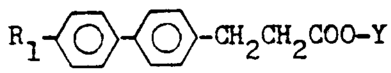

| $R_1$ | Y | K | $S_E$ | $S_B$ | $S_{I/F}$ | $S_C$ | $S_A$ | I |
|---|---|---|---|---|---|---|---|---|
| R8.0 | 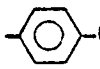-COO2MeBu (±) | | | 54 | 84 | 103 | 125 | |
| R8.0 | 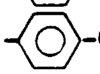-COO3MeBu (±) | | | 85 | 108 | 113 | 133 | |
| R8.0 | 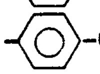-COOBu | | 98 | | | 112 | 138 | |
| R8.0 | 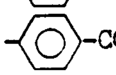-COO2Oct | | 71 | | 90 | 91 | 101 | |
| R8.0 | 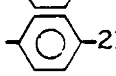-2MeBu (±) | | 65 | | 92 | 102 | 112 | |
| R8.0 | 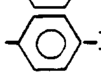-L-Et | | 72 | | | 91 | 109 | |
| R8.0 | 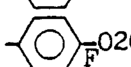O2Oct | | 91 | | | 97 | 100 | |
| R8.0 | 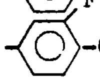-COO2Oct | | 40 | | 60 | 64 | 91 | |
| R8.0 | 2Oct | | | | | | 53 | |
| R9.0 | 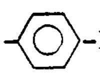-L-Et | | 42 | | 83 | 88 | 106 | |
| R9.0 | 2Oct | | | | | 72.4 | N 145.9 | |
| R10.0 | 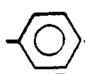-COO2Oct | 76 | 77 | | 89 | 94 | 96 | |
| R10.0 | 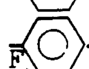-L-Et | | 44 | | 80 | 90 | 104 | |
| R9 | 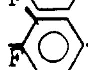$-C_9H_{19}$ | | | | 79 | | 85 | |
| R7.0 | 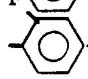$-C_9H_{19}$ | | | 62 | 66 | 99 | N 102 | |
| R8.0 | 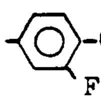-COO2Oct | 50 | | | 62.5 | 103 | 120 | |
| 2Oct | $C_8H_{17}$ | | | | | | 31 | |
| R8 | 2Oct | | | | | | 29 | |

Note: where the transition is to N rather than $S_A$ this is shown. 2MeBu = 2 methylbutyl, 3MeBu = 3 methylbutyl, Bu = butyl, -L-Et = $-COOC^{*}H(CH_3)COOC_2H_5$; 2Oct = $-C^{*}H(CH_3)C_6H_{13}$ (chiral).

The following ferroelectric smectic liquid crystal mixtures were prepared and investigated.

Mixture 1.

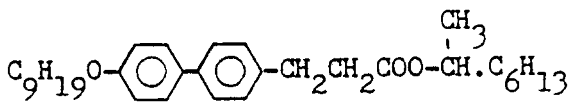

10 wt %

(optically active)

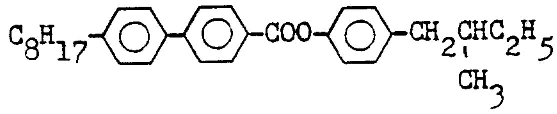

(±)

90 wt %

(racemic)

Transition temperatures (° C) $S_C^*$ 72.4 N 145.9 I

| Temperature (° C) | Extrapolated Ps (nC cm$^{-2}$) |
|---|---|
| 70 | 52 |
| 67.8 | 58 |
| 65.5 | 64 |
| 64 | 66 |
| 60 | 72 |
| 55 | 76 |
| (extrapolated Ps = Ps extrapolated to 100 wt% of the optically active compound). | |

Tilt angle of $S_C^*$ phase was ca 30°.

Mixture 2.

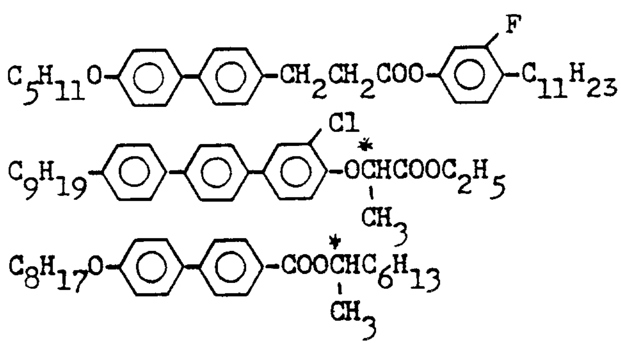

85.6 wt %

10.6 wt %

3.8 wt %

in this mixture the two optically active compounds induced opposite helical twist senses in the $S_C^*$ phase.

Transition temperatures:

K 49.5 $S_C$ 72.8 $S_A$ 75.8 N 86.8 I

| Temperature (°C) | Ps $nCcm^{-2}$ |
|---|---|
| 50 | 6.4 |
| 52.6 | 5.9 |
| 55 | 5.5 |
| 57.6 | 5.0 |
| 60 | 4.5 |
| 62.6 | 3.8 |
| 65.6 | 3.33 |
| 68 | 2.39 |
| 69 | 1.93 |
| 70 | 1.4 |
| 71 | 0.6 |
| 72.6 | 0.29 |

A log-log plot of response time against applied voltage at 60°C for this mixture in a 4 μm polyimide aligned cell are shown in fig 2.

Mixture 3

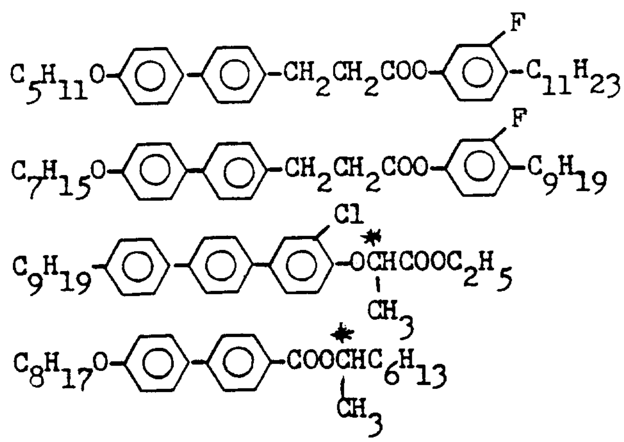

56.4 wt %

29 wt %

9.5 wt %

5.1 wt %

the two optically active compounds induced opposite helical twist senses in the $S_C^*$ phase. mixture 3 had transition temperatures:

K 40.5 $S_B$? 51.7 $S_C$ 75.6 $S_A$ 78.2 N 87.8 I

A graph of Ps against temperature for this mixture, and a log-log plot of response time against applied voltage are shown in fig, 3 and 4 respectively. Both measurements were made in 9 μm polyimide aligned cells.

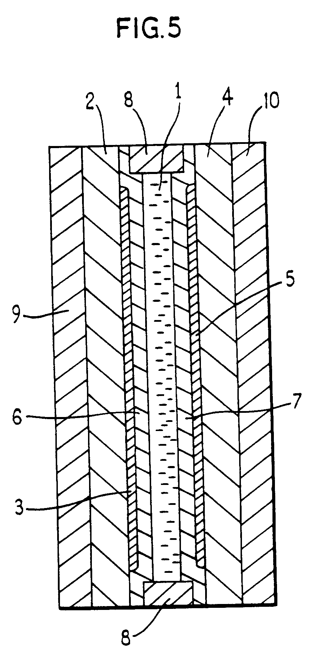

An example of the use of a compound of Formula I or II in a liquid crystal material and device embodying the present invention will now be described with reference to Figure 5.

In Figure 5 a liquid crystal cell comprises a layer 1 of liquid crystal material exhibiting a chiral smectic phase sandwiched between a glass slide 2 having a transparent conducting layer 3 on its surface, eg of tin oxide or indium oxide, and a glass slide 4 having a transparent conducting layer 5 on its surface. The slides 2,4 bearing the layers 3,5 are respectively coated by films 6,7 of a polyimide polymer. Prior to construction of the cell the films 6 and 7 are rubbed with a soft tissue in a given direction the rubbing directions being arranged parallel upon construction of the cell. A spacer 8 eg of polymethylmethacrylate, separates the slides 2,4 to the required distance, eg 5 microns. The liquid crystal material 1 is introduced between the slides 2,4 to the required distance, eg 5 microns. The liquid crystal material 1 is introduced between the slides 2,4 by filling the space between the slides 2, 4 and spacer 8 and sealing the spacer 8 in a vacuum in a known way. Preferably, the liquid crystal material in the smectic A or isotropic liquid phase (obtained by heating the material) when it is introduced beteen the slides 2,4 to facilitate alignment of the liquid crystal

molecules with the rubbing directions on the slides 2,4.

A polarizer 9 is arranged with its polarization axis parallel to the rubbing direction on the films 6,7 and an analyzer (crossed polarizer) 10 is arranged with its polarization axis perpendicular to that rubbing direction.

When a square wave voltage (from a conventional source not shown) varying between about +10 volts and -10 volts is applied across the cell by making contact with the layers 3 and 5 the cell is rapidly switched upon the change in sign of the voltage between a dark state and a light state as explained above.

In an alternative device (not shown) based on the cell construction shown in Figure 1 the layers 3 and 5 may be selectively shaped in a known way, eg by photoetching or deposition through a mask, eg to provide one or more display symbols, eg letters, numerals, words or graphics and the like as conventionally seen on displays. The electrode portions formed thereby may be addressed in a variety of ways which include multiplexed operation.

Mixture 4.

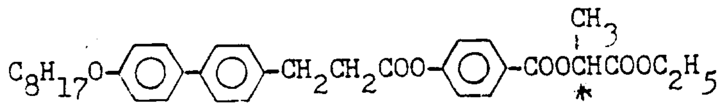

mixed with racemic:

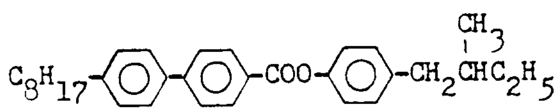

At 10 ° C below the $S_C$-$S_A$ transition the mixture had a Ps of 25 extrapolated to 100 % of the optically active compound.

Mixture 5.

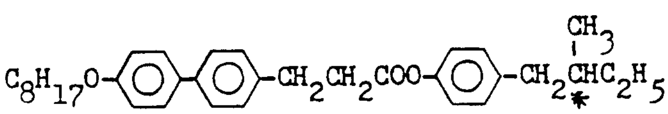

mixed with the racemic compound used in mixture 4. At 10 ° C below $S_C$-$S_A$ the Ps extrapolated to 100 % of the optically active compound was ≦ 0.4.

**Claims**

**1.** Use of a compound of Formula I for inclusion in a ferroelectric smectic liquid crystal mixture,where Formula I is:

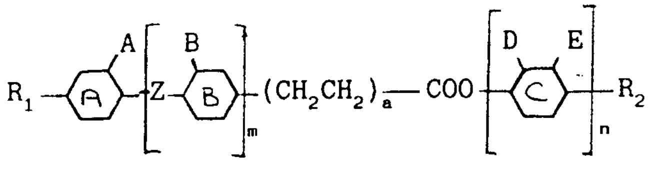

Formula I

where each of the figs A B and C are independently selected from phenyl, trans-cyclohexyl or pyrimidyl, n is 0 or 1, m is 1, 2 or 3 (provided m + n is 1, 2 or 3), each of A, B, D and E is independently hydrogen or fluorine, $R_1$ is alkyl or alkoxy containing up to 15 carbon atoms, Z is selected from COO, OOC, $CH_2CH_2$, $OCH_2$, $CH_2O$, CH=N, N=CH, N=N, N(O)=N or a single bond; and if n is 0, $R_2$ is alkyl containing up to 15 carbon atoms and if n is 1, $R_2$ is selected from -$COOR_3$ or

$-CH(CH_3)COOR_4$ where $R_3$ is optically active alkyl or n-alkyl and $R_4$ is n-alkyl and $R_3$ and $R_4$ contain up to 12 carbon atoms ; and a is 1 or 2.

**2.** A compound as defined in claim 1, having a formula IA;

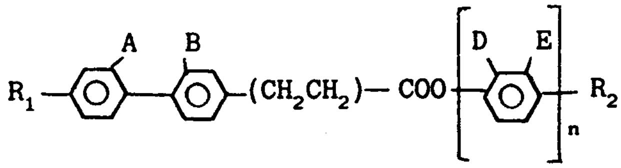

Formula IA

where A, B, D and E are either all hydrogen or else only one is fluorine.

**3.** A compound having a formula selected from formulae I.1 to I.7 below;

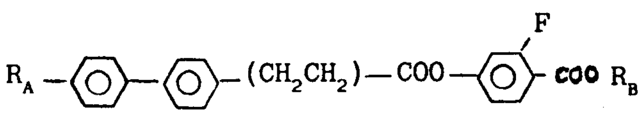

Formula I.1

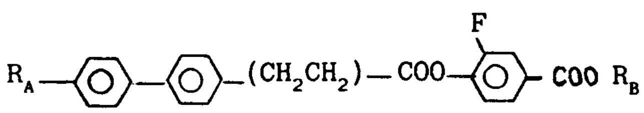

Formula I.2

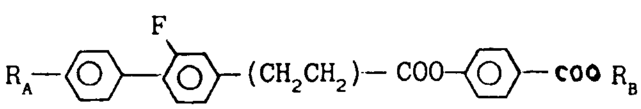

Formula I.3

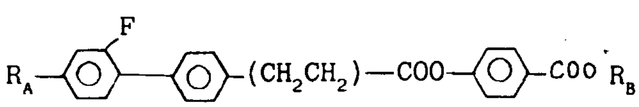

Formula I.4

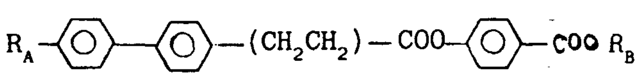

Formula I.5

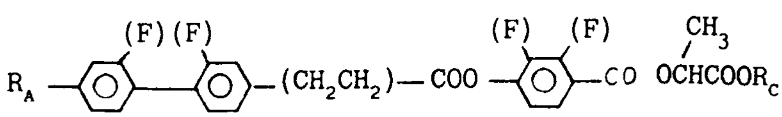

Formula I.6

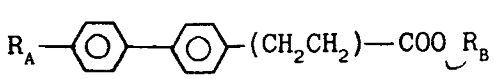

Formula I.7

where $R_A$ is n-alkyl or n-alkoxy or optically active alkyl or alkoxy. $R_B$ is alkyl, (F) indicates that the structure may contain one fluorine substituent at one of the substitution positions indicated. $R_C$ is n-alkyl and each of $R_A$ contains up to 15 carbon atoms, and $R_B$ and $R_C$ contains up to 15 carbon atoms.

**4.** A ferroelectric smectic liquid crystal mixture containing at least two compounds, at least one of which is a compound as defined in claim 1, 2 or 3.

**5.** A ferroelectric smectic liquid crystal mixture containing at least two compounds, at least one of which is a compound of formula II:

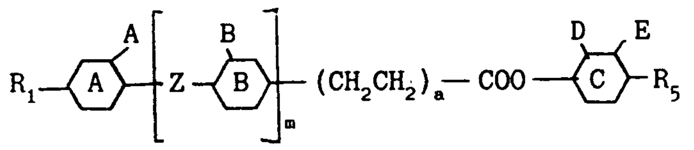

Formula II

where each of the figs A B and C are independently selected from phenyl, trans- cyclohexyl or pyrimidyl, each of A, B, D and E is independently hydrogen or fluorine, $R_1$ is alkyl or alkoxy containing up to 15 carbon atoms, Z is selected from COO, OOC, $CH_2CH_2$, $OCH_2$, $CH_2O$, CH=N, N=CH, N=N, N(O)=N or a single bond; a is 1 or 2, m is 1 or 2, and $R_5$ is n-alkyl, n-alkoxy, or optically active alkyl or alkoxy containing up to 15 carbon atoms.

6. A mixture as claimed in claim 5, wherein the compound of formula II has a formula IIA:

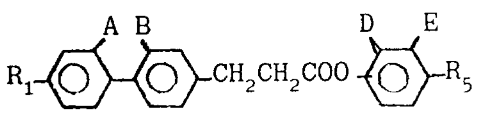

formula IIA

where either all of A, B, D and E are hydrogen or only one is fluorine.

7. A mixture as claimed in claim 6, wherein the compound of formula IIA has a formula:

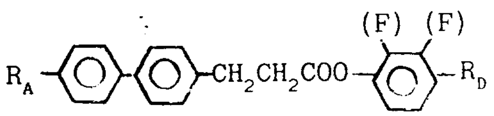

II.1

where $R_A$ is n-alkyl or n-alkoxy or optically active alkyl or alkoxy and $R_D$ is n-alkyl, n-alkoxy or optically active alkyl or alkoxy, and (F) indicates that the compound may carry one fluorine substituent at one of the substitution positions shown.

8. A mixture as claimed in claim 4,5, 6 or 7 wherein the compound of formula I or II is optically active.

9. A mixture as claimed in claim 4,5,6 or 7 wherein the compound of formula I or II is non-optically active or racemic.

10. A liquid crystal electro-optical display device which uses a ferroelectric smectic liquid crystal mixture as claimed in any one of claims 4 to 9.

**Patentansprüche**

1. Verwendung von Verbindungen als Komponente in einem ferroelektrischen smektischen Flüssigkristallgemisch, welche die Formel I besitzen,

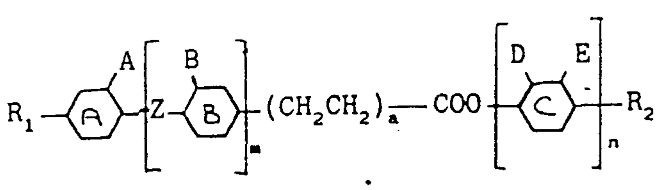

I,

worin bedeuten:

- Die Ringe

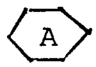 , 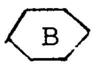 und 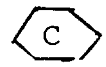

unabhängig Phenyl, trans-Cyclohexyl oder Pyrimidyl,
- A, B, D und E unabhängig Wasserstoff oder Fluor,
- $R_1$ Alkyl oder Alkoxy mit bis zu 15 C-Atomen,
- Z COO, OOC, $CH_2CH_2$, $OCH_2$, $CH_2O$, CH = N, N = CH, N = N, N(O) = N oder eine Einfachbindung,
- m 1, 2 oder 3,
- n 0 oder 1, wobei m + n gleich 1, 2 oder 3 ist,
- a 1 oder 2

  und
- $R_2$
  - Alkyl mit bis zu 15 C-Atomen, wenn n = O ist, und
- -$COOR_3$ oder -$CH(CH_3)COOR_4$, wenn n = 1 ist, wobei $R_3$ optisch aktives Alkyl oder n-Alkyl und

  $R_4$ n-Alkyl bedeuten und

  $R_3$ und $R_4$ bis zu 12 C-Atomen besitzen.

**2.** Verbindungen nach Anspruch 1 der Formel IA,

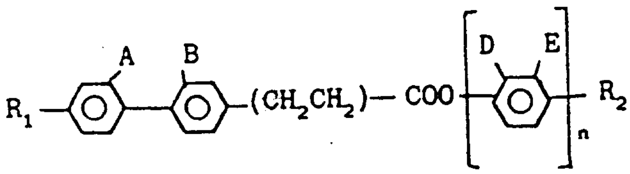 IA,

worin A, B, D und E entweder alle Wasserstoff sind oder nur einer dieser Substituenten Fluor bedeuten.

**3.** Verbindungen mit einer unter den Formeln I.1 bis I.7 ausgewählten Formel

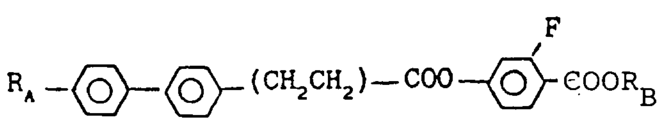 Formel I.1

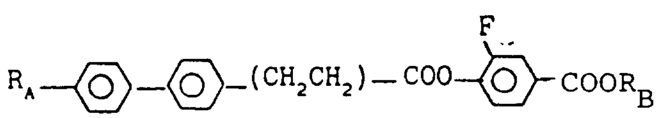 Formel I.2

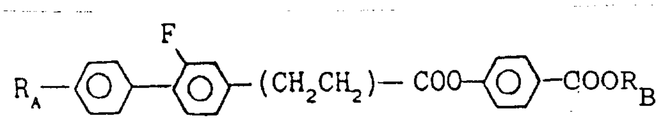 Formel I.3

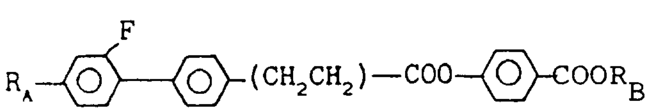 Formel I.4

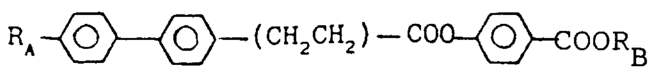

Formel I.5

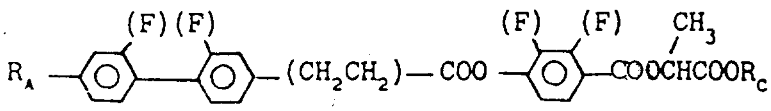

Formel I.6

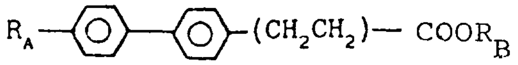

Formel I.7,

worin $R_A$ n-Alkyl oder n-Alkoxy oder optisch aktives Alkyl oder Alkoxy und $R_B$ Alkyl sind, (F) bedeutet, daß die Struktur an einer der angegebenen Substitutionsstellen einen Fluorsubstituenten enthalten kann, $R_C$ n-Alkyl ist und die Substituenten $R_A$ bis zu 15 Kohlenstoffatome und die Substituenten $R_B$ und $R_C$ bis zu 15 Kohlenstoffatome enthalten.

**4.** Ferroelektrisches smektisches Flüssigkristallgemisch, das mindestens zwei Verbindungen enthält, von denen mindestens eine eine Verbindung nach den Ansprüchen 1, 2 oder 3 ist.

**5.** Ferroelektrisches smektisches Flüssigkristallgemisch, das mindestens zwei Verbindungen enthält, von denen mindestens eine eine Verbindung der Formel II ist,

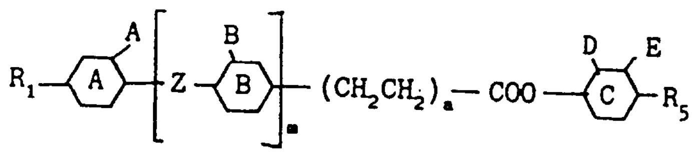

II,

worin bedeuten:

- Die Ringe

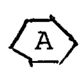 , 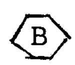 und 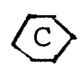

  unabhängig Phenyl, trans-Cyclohexyl oder Pyrimidyl,
- A, B, D und E unabhängig Wasserstoff oder Fluor,
- $R_1$ Alkyl oder Alkoxy mit bis zu 15 C-Atomen,
- Z COO, OOC, $CH_2CH_2$, $OCH_2$, $CH_2O$, CH = N, N = CH, N = N, N(O) = N oder eine Einfachbindung,
- m 1 oder 2,
- a 1 oder 2 und
- $R_5$ n-Alkyl, n-Alkoxy oder optisch aktives Alkyl oder Alkoxy mit bis zu 15 C-Atomen.

**6.** Flüssigkristallgemisch nach Anspruch 5, worin die Verbindung der Formel II die Formel IIA besitzt,

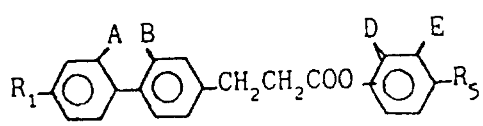

IIA,

worin entweder A, B, D und E alle Wasserstoff oder nur einer dieser Substituenten Fluor bedeuten.

7. Flüssigkristallgemisch nach Anspruch 6, worin die Verbindung der Formel IIA die Formel II.1 besitzt,

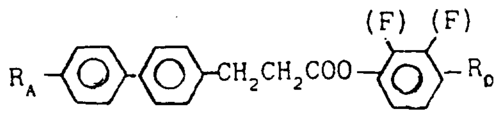

II.1,

worin $R_A$ n-Alkyl oder n-Alkoxy oder optisch aktives Alkyl oder Alkoxy und $R_D$ n-Alkyl, n-Alkoxy oder optisch aktives Alkyl oder Alkoxy sind und (F) bedeutet, daß die Verbindung an einer der angegebenen Substitutionsstellen einen Fluorsubstituenten enthalten kann.

8. Flüssigkristallgemisch nach Anspruch 4, 5, 6 oder 7, worin die Verbindung der Formel I oder II optisch aktiv ist.

9. Flüssigkristallgemisch nach Anspruch 4, 5, 6 oder 7, worin die Verbindung der Formel I oder II nicht optisch aktiv oder racemisch ist.

10. Elektrooptische Flüssigkristallanzeigevorrichtung, in der ein ferroelektrisches smektisches Flüssigkristallgemisch nach einem der Ansprüche 4 bis 9 verwendet ist.

**Revendications**

1. Utilisation d'un composé de formule I pour une inclusion dans un mólange ferrosmootique à propriétés de cristaux liquides, cette formule I étant :

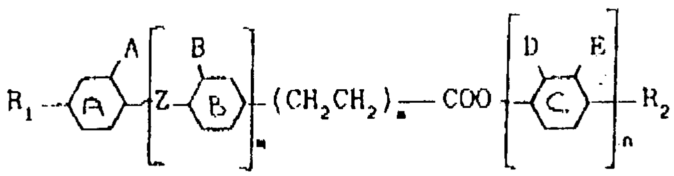

Formule I

dans laquelle chacun des noyaux A, B et C est choisi, indépendamment, parmi un noyau phényle, trans-cyclohexyle ou pyrimidyle ; n vaut 0 ou 1 ; m vaut 1, 2 ou 3 (à la condition que la somme (m + n) vale 1, 2 ou 3)), chacun des symboles A, B, D et E (des substituants) représente indépendamment, un atome d'hydrogène ou de fluor ; $R_1$ représente un groupe alkyle ou alcoxy contenant jusqu'à 15 atomes de carbone ; Z est choisi parmi COO, OOC, $CH_2CH_2$, $OCH_2$, $CH_2O$, CH = N, N = CH, N = N, N-(O) = N ou une liaison simple ; et, si n est nul, $R_2$ représente un groupe alkyle contenant jusqu'à 15 atomes de carbone et si n vaut 1, $R_2$ est choisi parmi un groupe $-COOR_3$ ou $-CH(CH_3)COOR_4$, formules dans lesquelles $R_3$ représente un groupe alkyle optiquement actif ou un groupe n-alkyle, et $R_4$ représente un groupe n-alkyle, et $R_3$ et $R_4$ contiennent jusqu'à 12 atomes de carbone ; et a vaut 1 ou 2.

2. Composé tel que défini à la revendication 1, ayant pour formule 1A :

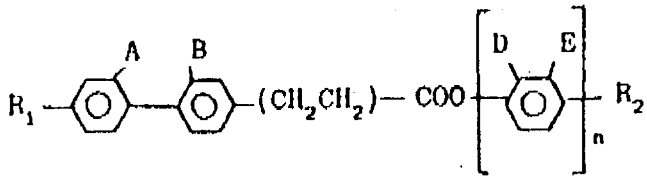

Formule IA

dans laquelle les symboles A, B, D et E (des substituants) représentent tous des atomes d'hydrogène, ou bien un seul d'entre eux représente un atome de fluor.

**3.** Composé ayant une formule choisie parmi les formules I.1 à I.7 ci-dessous :

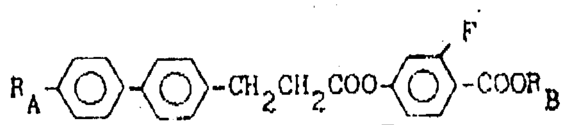
I.1

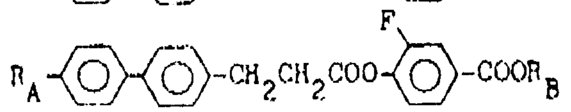
I.2

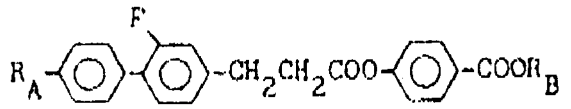
I.3

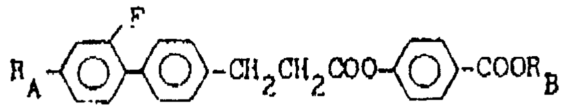
I.4

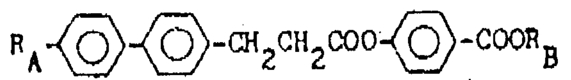
I.5

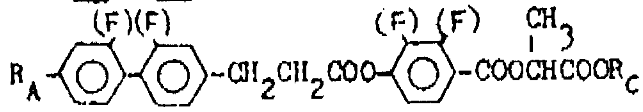
I.6

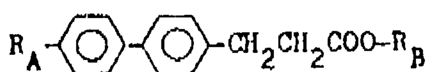
I.7

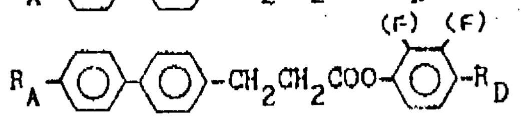
II.1

formules dans lesquelles $R_A$ représente un groupe n-alkyle ou n-alcoxy ou un groupe alkyle ou alcoxy optiquement actif; $R_B$ représente un groupe alkyle ; (F) indique que la structure peut contenir un substituant fluor sur l'une des positions de substitution indiquées ; $R_C$ représente un groupe n-alkyle et chacun des groupes $R_A$ contient jusqu'à 15 atomes de carbone et $R_B$ et $R_C$ contiennent jusqu'à 15 atomes de carbone.

**4.** Mélange smectique ferro-électrique à propriétés de cristaux liquides, contenant au moins deux composés, dont l'un au moins est un composé tel que défini à la revendication 1, 2 ou 3.

**5.** Mélange smectique ferro-électrique à propriétés de cristaux liquides, contenant au moins deux composés, dont l'un au moins est un composé de formule II :

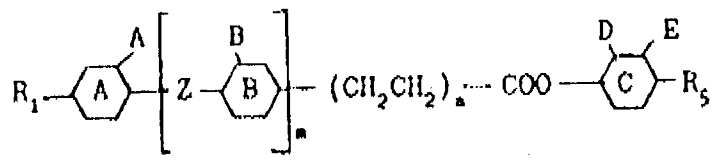
Formule II

dans laquelle chacun des noyaux A, B et C est choisi, indépendamment, parmi un noyau phényle, trans-cyclohexyle ou pyrimidyle ; chacun des symboles A, B, D et E (des substituants) représente, indépendamment, un atome d'hydrogène ou de fluor, $R_1$ représente un groupe alkyle ou alcoxy contenant jusqu'à 15 atomes de carbone ; Z est choisi parmi, COO, OOC, $CH_2CH_2$, $OCH_2$, $CH_2O$, CH = N, N = CH, N = N, N(O) = N ou une liaison simple ; a vaut 1 ou 2 ; n vaut 1 ou 2; et $R_5$ représente un groupe n-alkyle, n-alcoxy ou un groupe alkyle ou alcoxy optiquement actif contenant jusqu'à 15 atomes de carbone.

6. Mélange tel que revendiqué à la revendication 5, dans lequel le composé de formule II a une formule IIA

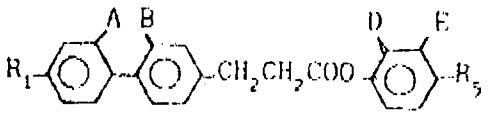

Formule IIA

dans laquelle les symboles A, B, D et E (des substituants) représentent chacun un atome d'hydrogène, ou bien un seul de ces symboles représente un atome de fluor.

7. Mélange tel que revendiqué à la revendication 6, dans lequel le composé de formule IIA a pour formule

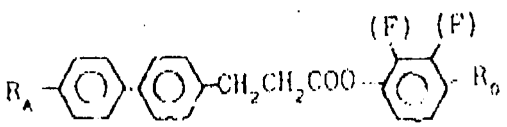

II.1

dans laquelle $R_A$ représente un groupe n-alkyle ou n- alcoxy ou un groupe alkyle ou alcoxy optiquement actif, et $R_B$ représente un groupe n-alkyle, n-alcoxy ou un groupe alkyle ou alcoxy optiquement actif, et (F) indique que le composé peut porter un substituant fluor sur l'une des positions de substitution montrées.

8. Mélange tel que revendiqué à la revendication 4, 5, 6 ou 7, dans lequel le composé de formule I ou II est optiquement actif.

9. Mélange tel que revendiqué à la revendication 4, 5, 6 ou 7, dans lequel le composé de formule I ou II n'est pas optiquement actif ou est racémique.

10. Dispositif d'affichage électro-optique à cristaux liquides, qui utilise un mélange smectique ferroélectrique à propriétés de cristaux liquides, tel que revendiqué dans l'une quelconque des revendications 4 à 9.

FIG.1

ROUTE A

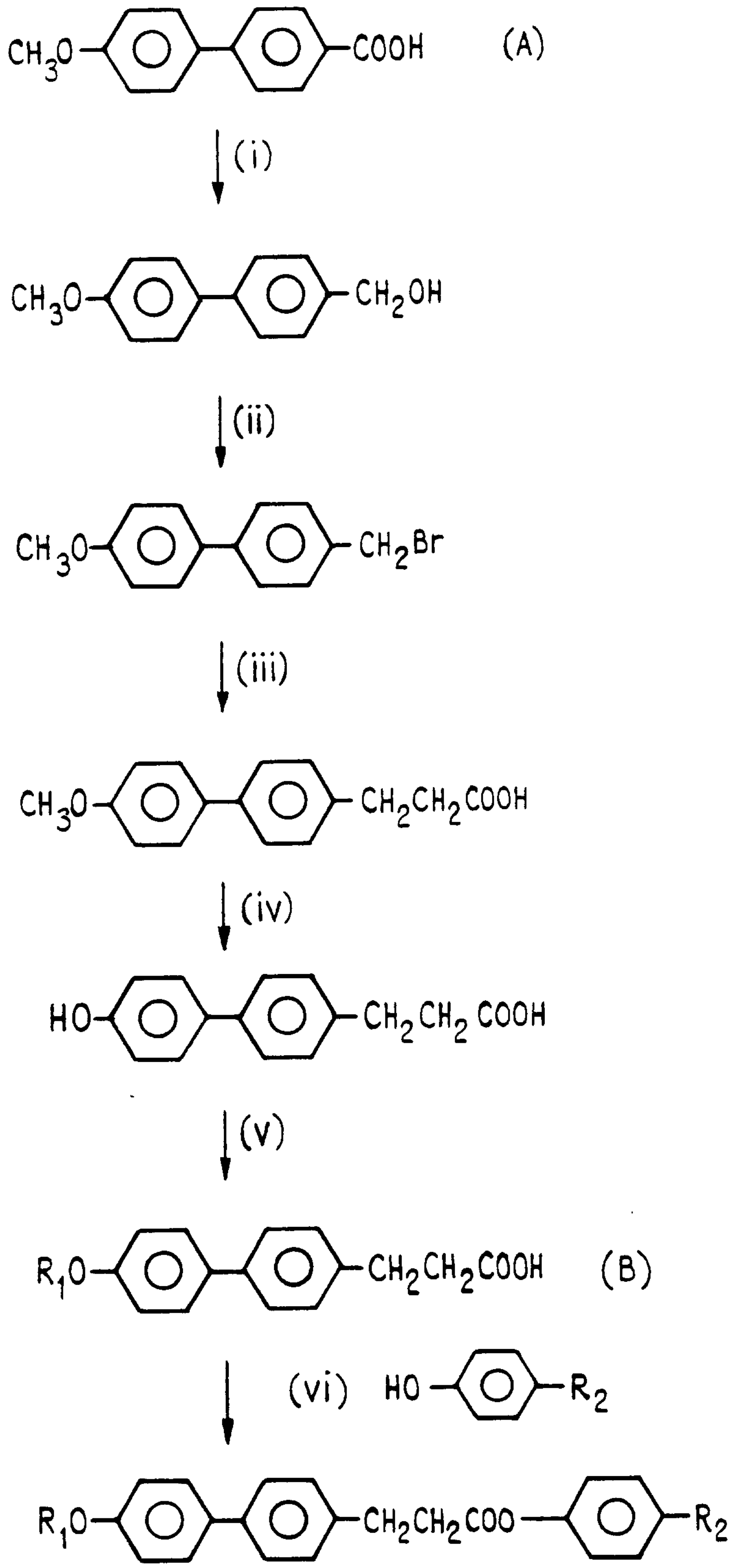

FIG. 2

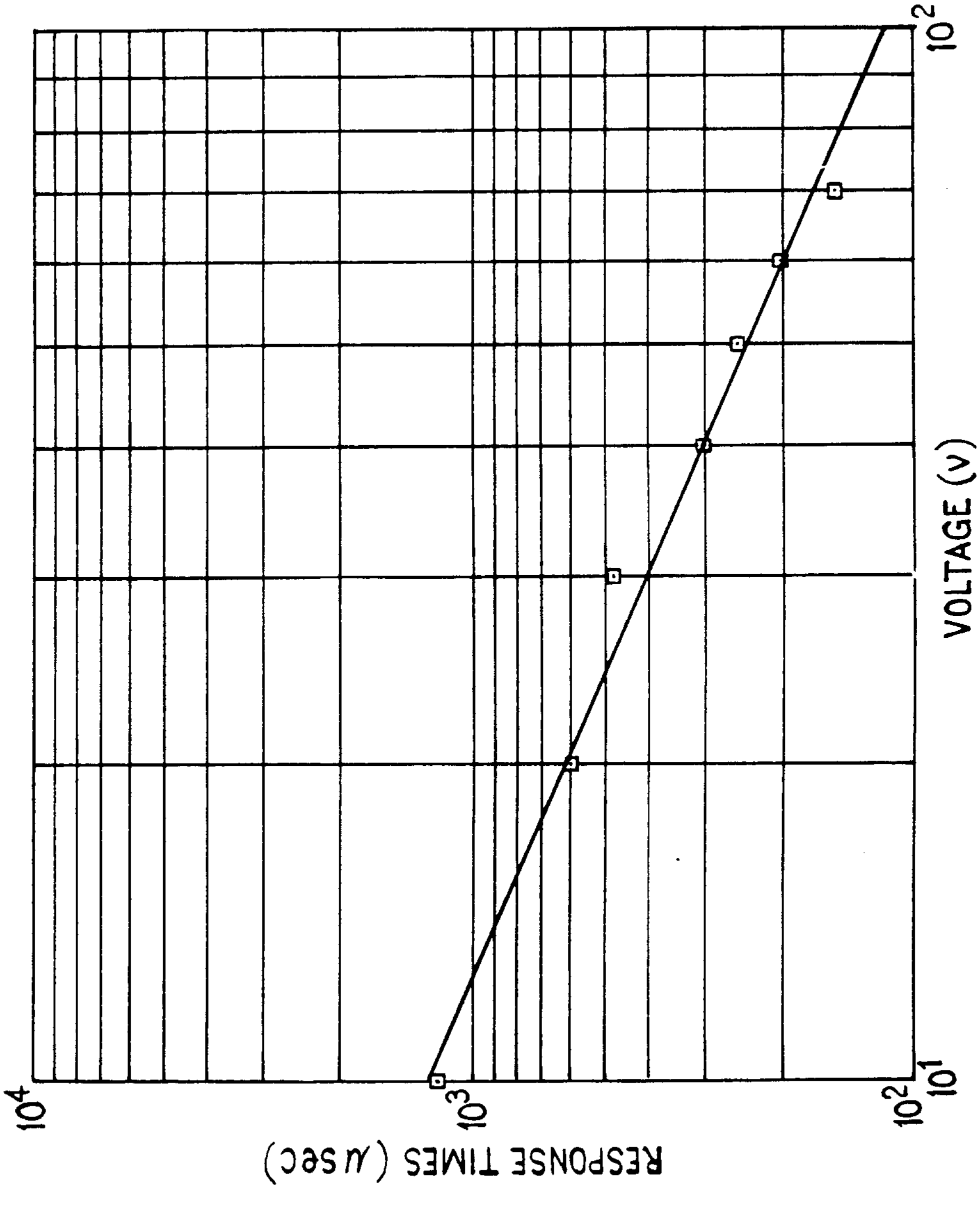

FIG. 3

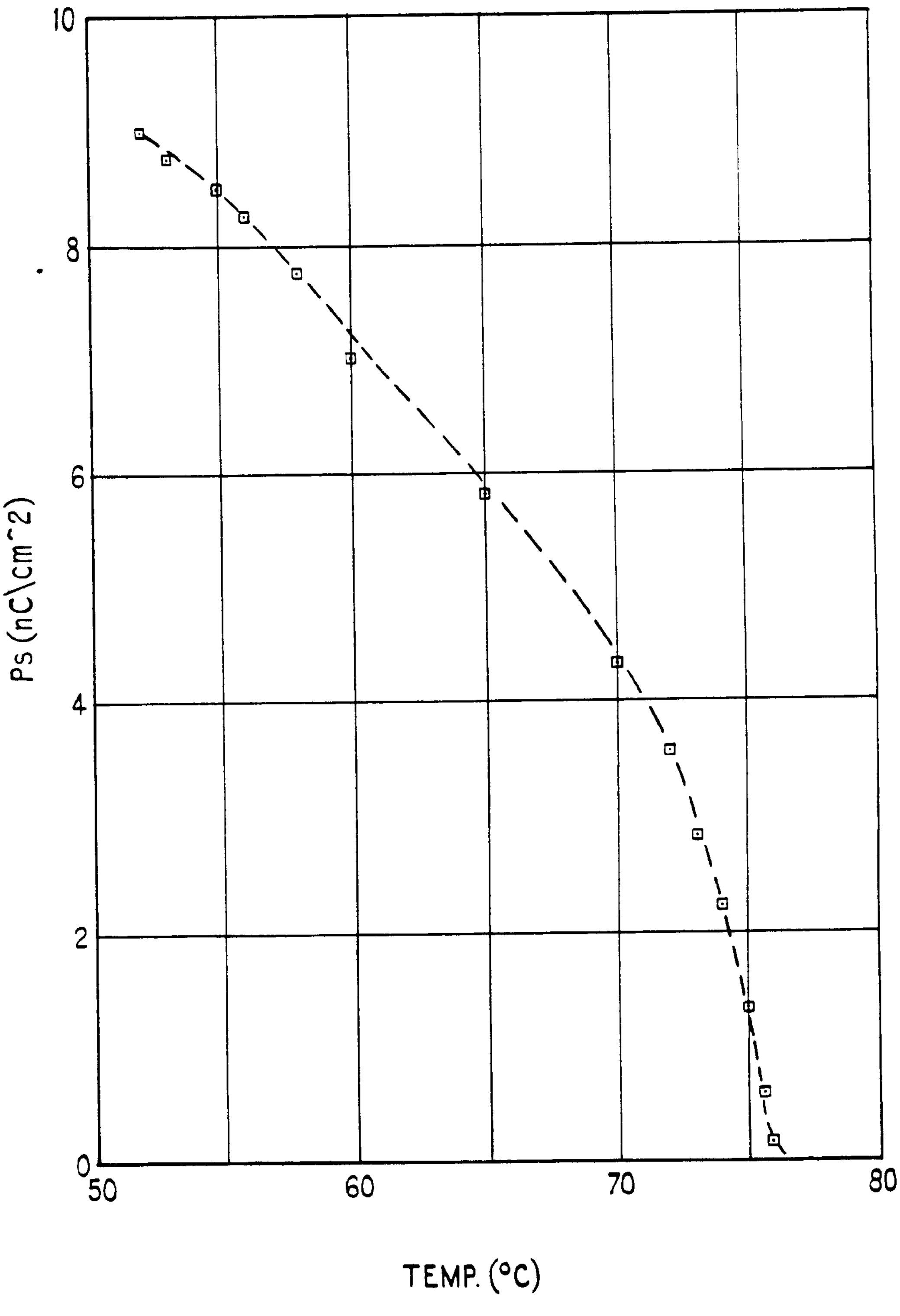

FIG.4

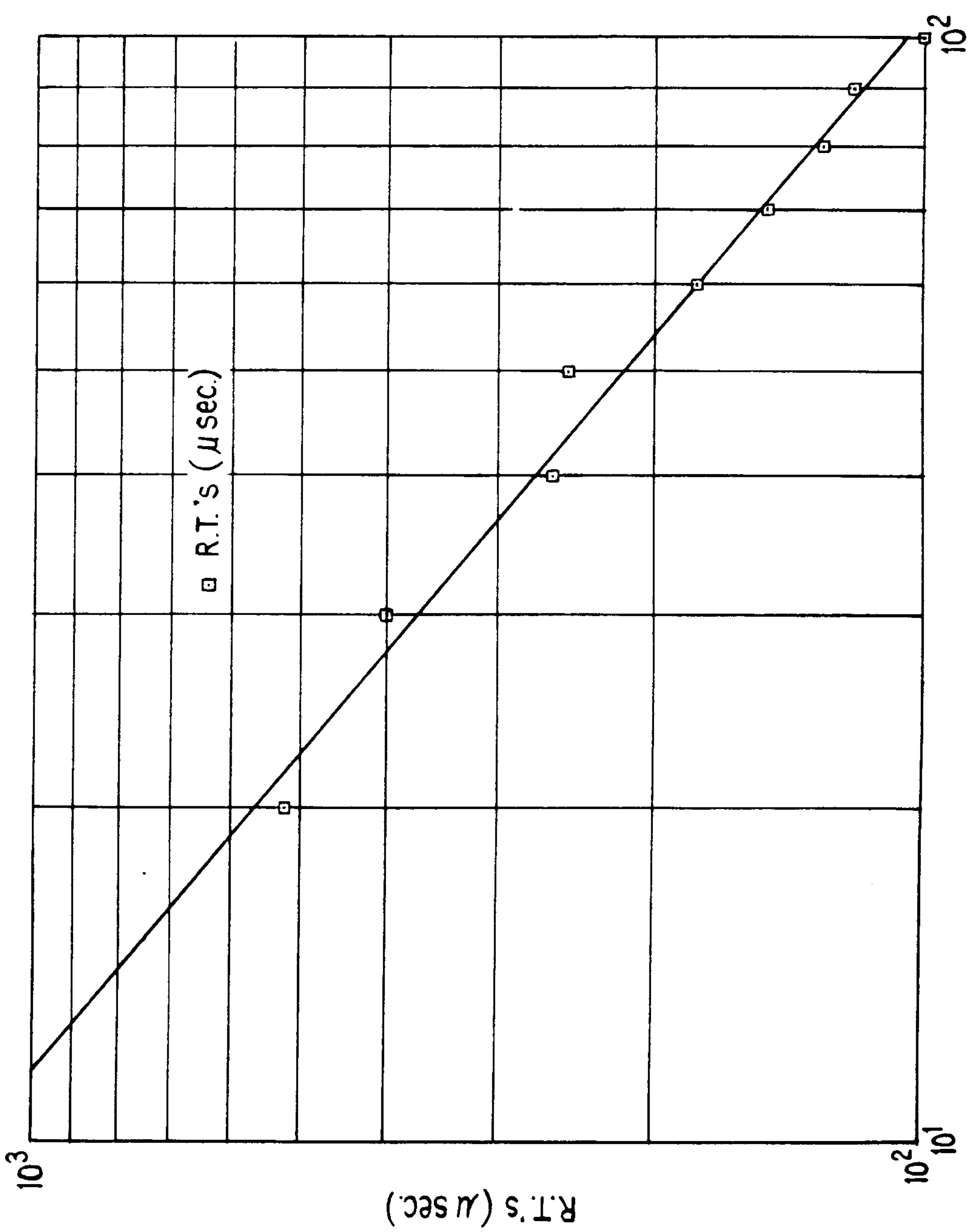

FIG.5